# EUROPÄISCHE PATENTSCHRIFT

(11) **EP 3 440 203 B1**
(45) Veröffentlichungstag und Bekanntmachung des Hinweises auf die Patenterteilung: **29.11.2023**
(21) Anmeldenummer: 17715682.5
(22) Anmeldetag: 04.04.2017
(51) Int. Cl.: C12N 9/48, C11D 3/386, C12N 9/54

(54) **NEUE PROTEASE MIT VERBESSERTER WASCHLEISTUNG**
NOVEL PROTEASE WITH IMPROVED WASHING PERFORMANCE
NOUVELLE PROTÉASE À PERFORMANCE DE LAVAGE AMÉLIORÉE

(30) Priorität: 06.04.2016 DE 102016205670
(43) Veröffentlichungstag der Anmeldung: 13.02.2019
(73) Patentinhaber: Henkel AG & Co. KGaA, 40589 Düsseldorf (DE)
(72) Erfinder: HERBST, Daniela, 40237 Düsseldorf (DE); O'CONNELL, Timothy, 86899 Landsberg am Lech (DE); MUSSMANN, Nina, 47877 Willich (DE); LAUFS, Brian, 41363 Jüchen (DE); AYDEMIR, Ayhan, 40589 Düsseldorf (DE); EICHSTÄDT, Renée Charlott, 50733 Köln (DE); PRÜSER, Inken, 40215 Düsseldorf (DE)
(86) Internationale Anmeldenummer: PCT/EP2017/057907
(87) Internationale Veröffentlichungsnummer: WO 2017/174536

(56) Entgegenhaltungen:
- WO-A2-2009/149144
- WO-A2-2009/149200
- WO-A2-2010/056653
- WO-A2-2011/072099
- PAUL CARTER ET AL: "Probing the mechanism and improving the rate of substrate-assisted catalysis in subtilisin BPN'", BIOCHEMISTRY, Bd. 30, Nr. 25, 25. Juni 1991 (1991-06-25) , Seiten 6142-6148, XP055377697, US ISSN: 0006-2960, DOI: 10.1021/bi00239a009

## Beschreibung

Die Erfindung liegt auf dem Gebiet der Enzymtechnologie. Die Erfindung betrifft eine Protease aus *Bacillus amyloliquefaciens,* deren Aminosäuresequenz, insbesondere im Hinblick auf den Einsatz in Wasch- und Reinigungsmitteln verändert wurde, um eine verbesserte Reinigungsleistung zu erhalten, und die für sie kodierenden Nukleinsäuren sowie deren Herstellung. Die Erfindung betrifft ferner die Verwendungen dieser Protease und Verfahren, in denen sie eingesetzt wird, sowie diese enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus-*Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

Besonders bevorzugte Subtilisine, die kommerziell in Waschmitteln eingesetzt werden sind die beiden Proteasen Subtilisin Carlsberg und eine Variante ausgehend von der *Bacillus lentus* DSM 5483 alkalischen Protease (BLAP), die ausgehend vom BLAP Wildtyp die Mutationen S3T, V4I, V193M, V199I und L211D in der Zählung gemäß BLAP aufweist (Referenzprotease 1). Aus WO 2011/072099, WO 2009/149144, WO 2009/149200 und WO 2010/056653 sind verschiedene Bacillus amyloliquefaciens Protease (BPN') Varianten bekannt.

Überraschenderweise wurde jetzt festgestellt, dass eine Protease des Subtilisin-Typs aus *Bacillus amyloliquefaciens* oder eine hierzu hinreichend ähnliche Protease (bezogen auf die Sequenzidentität), die die Aminosäuresubstitutionen an den Positionen S24G, S33T, S53G, S78N, S101N, G128A und L217Q, jeweils bezogen auf die Nummerierung gemäß SEQ ID NO:1, aufweist, im Vergleich zu Proteasen des Standes der Technik, insbesondere Referenzprotease 1 und 2 (BLAP Wildtyp die Mutationen S3T, V4I, V193M, V199I und L211D in der Zählung gemäß BLAP und Subtilisin Carlsberg) eine verbesserte Reinigungsleistung in Bezug auf verschiedene Protein-haltige Anschmutzungen aufweist und daher besonders für den Einsatz in Wasch- oder Reinigungsmitteln geeignet ist.

Gegenstand der Erfindung ist daher in einem ersten Aspekt eine Protease umfassend eine Aminosäuresequenz wie in SEQ ID NO:2 angegeben.

Ein weiterer Gegenstand der Erfindung ist ein Verfahren zur Herstellung einer solchen Protease, umfassend das Substituieren der Aminosäuren an den Positionen, die Positionen 24, 33, 53, 78, 101, 128 und 217 in SEQ ID NO:1 entsprechen, in einer Ausgangsprotease mit einer wie in SEQ ID NO:1 angegebenen Aminosäuresequenz derart, dass die Protease an den entsprechenden Positionen die Aminosäuren 24G, 33T, 53G, 78N, 101N, 128A und 217Q umfasst.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als Stoff wie auch auf die entsprechenden Verfahren, insbesondere Herstellungsverfahren der Protease.

Weitere Aspekte der Erfindung betreffen die für diese Proteasen kodierenden Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren, und Verwendungen der erfindungsgemäßen Proteasen in Wasch- oder Reinigungsmitteln zur Entfernung von protein- oder peptidhaltigen Anschmutzungen.

"Mindestens eine", wie hierin verwendet, bedeutete eine oder mehrere, d.h. 1, 2, 3, 4, 5, 6, 7, 8, 9, 10, 11, 12, 13, 14 oder mehr.

Die vorliegende Erfindung basiert auf der überraschenden Erkenntnis der Erfinder, dass eine erfindungsgemäße Protease gemäß SEQ ID NO:2 im Vergleich zu den Referenzproteasen 1 und 2 eine verbesserte Reinigungsleistung in Bezug auf verschiedene proteinhaltige Anschmutzungen aufweist. Das ist insbesondere insoweit überraschend, als dass die Referenzproteasen 1 und 2 kommerziell in Waschmitteln eingesetzt werden und für diese Aufgabe optimiert wurden.

Die erfindungsgemäße Proteasen verfügt über eine besondere Stabilität in Wasch- oder Reinigungsmitteln, beispielsweise gegenüber Tensiden und/oder Bleichmitteln und/oder gegenüber Temperatureinflüssen, insbesondere gegenüber hohen Temperaturen, beispielsweise zwischen 30°C und 55°C, insbesondere 40°C, und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. Solche leistungsverbesserten Proteasen ermöglichen verbesserte Waschergebnisse an protease-sensitiven Anschmutzungen in einem weiten Temperaturbereich.

Eine erfindungsgemäße Protease weist eine proteolytische Aktivität auf, das heißt, sie ist zur Hydrolyse von Peptidbindungen eines Polypeptids oder Proteins befähigt, insbesondere in einem Wasch- oder Reinigungsmittel. Eine erfindungsgemäße Protease ist daher ein Enzym, welches die Hydrolyse von Peptidbindungen katalysiert und dadurch in der Lage ist, Peptide oder Proteine zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife Enzyme.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

In einer weiteren Ausführungsform der Erfindung ist die Protease dadurch gekennzeichnet, dass ihre Reinigungsleistung gegenüber derjenigen einer Protease, die eine Aminosäuresequenz umfasst, die der in SEQ ID NO:1 angegebenen Aminosäuresequenz entspricht, nicht signifikant verringert ist, d.h. mindestens 80 % der Referenzwaschleistung besitzt, vorzugsweise mindestens 100 %, noch bevorzugter mindestens 110%. Die Reinigungsleistung kann in einem Waschsystem bestimmt werden, das ein Waschmittel in einer Dosierung zwischen 4,5 und 7,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 60 Minuten bei einer Temperatur von 40°C erfolgen und das Wasser eine Wasserhärte zwischen 15,5 und 16,5° (deutsche Härte) aufweisen. Die Konzentration der Protease in dem für dieses Waschsystem bestimmten Waschmittel beträgt 0,0001-1,0 Gew.-%, vorzugsweise 0,006 bis 0,6 Gew.-%, bezogen auf aktives, gereinigtes Protein.

Ein flüssiges Referenzwaschmittel für ein solches Waschsystem kann wie folgt zusammengesetzt sein (alle Angaben in Gewichts-Prozent): 7% Alkylbenzolsulfonsäure, 9% weitere anionische Tenside, 4% C12-C18 Na-Salze von Fettsäuren (Seifen), 7% nicht-ionische Tenside, 0,7% Phosphonate, 3,2% Zitronensäure, 3,0% NaOH, 0,04% Entschäumer, 5,7% 1,2-Propandiol, 0,1% Konservierungsstoffe, 2% Ethanol, 0,2% Farbstoff-Transfer-Inhibitor, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird in einem pH-Wertebereich zwischen pH 8 und pH 10,5, bevorzugt zwischen pH 8 und pH 9 gewaschen.

Im Rahmen der Erfindung erfolgt die Bestimmung die Reinigungsleistung bei 40°C unter Verwendung eines flüssigen Waschmittels wie vorstehend angegeben, wobei der Waschvorgang vorzugsweise für 60 Minuten erfolgt.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann.

Verfahren zur Bestimmung der Proteaseaktivität sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

Ein alternativer Test zur Feststellung der proteolytischen Aktivität der erfindungsgemäßen Protease ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913) erfolgen.

Zusätzlich zu den vorstehend erläuterten Aminosäureveränderungen können Proteasen weitere Aminosäureveränderungen, insbesondere Aminosäure-Substitutionen, -Insertionen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Protease noch weiter erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt S24G die Substitution von Serin an Position 24 durch Glycin, S24AG die Insertion von Glycin nach der Aminosäure Alanin an Position 24 und S24* oder ΔS24 die Deletion von Serin an Position 24. Diese Nomenklatur ist dem Fachmann auf dem Gebiet der Enzymtechnologie bekannt.

Die Protease kann dadurch gekennzeichnet sein, dass sie aus einer Protease wie vorstehend beschrieben als Ausgangsmolekül erhältlich ist durch ein- oder mehrfache konservative Aminosäuresubstitution, wobei die Protease in der Zählung gemäß SEQ ID NO:1 noch die erfindungsgemäßen Aminosäuresubstitutionen an den Positionen, die Positionen 24, 33, 53, 78, 101, 128 und 217 in SEQ ID NO:1 entsprechen, aufweist, wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen umfassen beispielsweise: G=A=S, I=V=L=M, D=E, N=Q, K=R, Y=F, S=T, G=A=I=V=L=M=Y=F=W=P=S=T.

Alternativ oder ergänzend kann die Protease dadurch gekennzeichnet sein, dass sie aus einer Protease als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270 oder 275 zusammenhängenden Aminosäuren mit dem Ausgangsmolekül übereinstimmt, wobei die im Ausgangsmolekül enthaltenen mutierten Aminosäurereste an den Positionen, die Positionen 24, 33, 53, 78, 101, 128 und 217 in SEQ ID NO:1 entsprechen, noch vorhanden sind.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80%, vorzugsweise mindestens 90 % der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Die weiteren Aminosäurepositionen werden hierbei durch ein Alignment der Aminosäuresequenz einer Protease mit der Aminosäuresequenz der Protease aus *Bacillus amyloliquefaciens,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer Protease eine höhere Zahl von Aminosäureresten umfasst als die Protease aus *Bacillus amyloliquefaciens* gemäß SEQ ID NO:1. Ausgehend von den genannten Positionen in der Aminosäuresequenz der Protease aus *Bacillus amyloliquefaciens* sind die Veränderungspositionen in einer Protease diejenigen, die eben diesen Positionen in einem Alignment zugeordnet sind.

Vorteilhafte Positionen für Sequenzveränderungen, insbesondere Substitutionen, der Protease aus *Bacillus amyloliquefaciens,* die übertragen auf homologe Positionen der erfindungsgemäßen Proteasen bevorzugt von Bedeutung sind und der Protease vorteilhafte funktionelle Eigenschaften verleihen, sind demnach die Positionen 24, 33, 53, 78, 101, 128 und 217, zuzuordnen in einem Alignment mit SEQ ID NO:1 und damit in der Zählung gemäß SEQ ID NO:1. In den genannten Positionen liegen in der Referenzprotease aus *Bacillus amyloliquefaciens* folgende Aminosäurereste: S24, S33, S53, S78, S101, G128 und L217.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Protease aus *Bacillus amyloliquefaciens* gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar.

Demnach kann ein Verfahren ferner einen oder mehreren der folgenden Verfahrensschritte umfassen:
(a) Einbringen einer ein- oder mehrfachen konservativen Aminosäuresubstitution, wobei die Protease in der Zählung gemäß SEQ ID NO:1 die Aminosäuresubstitutionen 24G, 33T, 53G, 78N, 101N, 128A und 217Q aufweist;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270 oder 275 zusammenhängenden Aminosäuren mit dem Ausgangsmolekül übereinstimmt, wobei die im Ausgangsmolekül enthaltenen Aminosäuresubstitutionen 24G, 33T, 53G, 78N, 101N, 128A und 217Q noch vorhanden sind;

Sämtliche Ausführungen gelten auch für die erfindungsgemäßen Verfahren.

Die erfindungsgemäße Protease beziehungsweise die mit einem erfindungsgemäßen Verfahren hergestellte Protease weist die Aminosäuresubstitutionen 24G, 33T, 53G, 78N, 101N, 128A und 217Q auf.

Eine zuvor beschriebene Protease kann zusätzlich stabilisiert sein, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt. So können Proteasen beispielsweise auch dadurch stabilisiert werden, dass einer oder mehrere Tyrosin-Reste gegen andere Aminosäuren ausgetauscht werden.

Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein Protease kann auch mindestens eine chemische Modifikation aufweisen. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit Protease-Inhibitoren ist diesbezüglich möglich.

Betreffend alle vorstehend beschriebenen Proteasen beziehungsweise Proteasevarianten und/oder Derivate sind diejenigen besonders bevorzugt, deren Stabilität und/oder Aktivität mindestens derjenigen der Protease gemäß SEQ ID NO:2 entspricht, und/oder deren Reinigungsleistung mindestens derjenigen der Protease gemäß SEQ ID NO:2 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease kodiert, sowie ein Vektor enthaltend eine solche Nukleinsäure, insbesondere ein Klonierungsvektor oder ein Expressionsvektor.

Hierbei kann es sich DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren kodieren können, so dass eine bestimmte Aminosäuresequenz von mehreren unterschiedlichen Nukleinsäuren kodiert werden kann. Auf Grund dieser Degeneriertheit des genetischen Codes sind sämtliche Nukleinsäuresequenzen in diesen Erfindungsgegenstand mit eingeschlossen, die eine erfindungsgemäße Proteasen kodieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz kodierende Nukleinsäuren problemlos ermitteln. Weiterhin können bei erfindungsgemä-ßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure kodierend führt das dazu, dass in dem Organismus ein Codon weniger effizient translatiert wird als ein synonymes Codon, das für dieselbe Aminosäure kodiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder-Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemä-ßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von E*scherichia, Klebsiella, Bacillus, Staphylococcus, Corynebakterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotischen Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um eine erfindungsgemäße Protease herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Protease. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Proteasen herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

In verschiedenen Ausführungsformen enthält das erfindungsgemäße Mittel anionische Tenside. Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen, insbesondere C12-C18 Fettsäuren-Natrium-Salze. Diese sind bevorzugt zwischen 1 Gew.-% bis 7 Gew.-%, besonders bevorzugt zwischen 3 Gew.-% bis 5 Gew.-% im Mittel enthalten. Im Allgemeinen können Fettsäuren auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen. Die Tenside vom Sulfonat-Typ sind bevorzugt zwischen 4 Gew.-% bis 10 Gew.-%, besonders bevorzugt zwischen 6 Gew.-% bis 8 Gew.-% im Mittel enthalten.

Ferner kann das Mittel nicht-ionische Tenside enthalten. Für Wasch- und Reinigungsmittel geeignete nicht-ionische Tenside sind aus der internationalen Patentanmeldung WO2009/121725 bekannt. Im erfindungsgemäßen Mittel sind sie bevorzugt zwischen 6 Gew.-% bis 12 Gew.-%, besonders bevorzugt zwischen 8 Gew.-% bis 10 Gew.-% enthalten.

Zusätzlich kann das hierin beschriebene Mittel mindestens einen Builder enthalten. Bekannte Builder sind in WO2009/121725 offenbart. Im erfindungsgemäßen Mittel sind die bevorzugten Builder Zitronensäure/Citrat und/oder Phosphonate. Citrat/Zitronensäure ist bevorzugt zwischen 0,5 Gew.-% bis 6 Gew.-%, besonders bevorzugt zwischen 2 Gew.-% bis 4 Gew.-% im Mittel enthalten. Der bevorzugte Phosphonat-Gehalt im erfindungsgemäßen Mittel ist zwischen 0,4 Gew.-% bis 1,0 Gew.-% und besonders bevorzugt zwischen 0,6 Gew.-% bis 0,8 Gew.-%.

Zur Stabilisierung der Enzyme kann das Mittel ferner bekannte Stabilisatoren enthalten. In diesem Zusammenhang wird die Verwendung von 1,2-Propandiol bevorzugt. 1,2-Propandiol kann zwischen 3 Gew.-% bis 9 Gew.-%, bevorzugt zwischen 5 Gew.-% bis 7 Gew.-% im erfindungsgemä-ßen Mittel enthalten sein.

Ein erfindungsgemäßes Mittel enthält die Protease vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäß besonders bevorzugt sind Waschmittel, insbesondere Flüssigwaschmittel. Solche Flüssigwaschmittel können zusätzlich zu der Protease anionische Tenside, insbesondere lineare Alkylbenzolsulfonate, nicht-ionische Tenside, und mindestens einen Builder umfassen. In einer Ausführungsform der Erfindung umfasst ein solches Flüssigwaschmittel jeweils bezogen auf das Gesamtgewicht des Mittels:
a) 4 Gew.-% bis 10 Gew.-%, bevorzugt 6 Gew.-% bis 8 Gew.-% eines linearen Alkylbenzolsulfonats;
b) 6 Gew.-% bis 12 Gew.-%, bevorzugt 8 Gew.-% bis 10 Gew.-% eines weiteren anionischen Tensids, insbesondere eine Alkylethersulfats;
c) 0,5 Gew.-% bis 6 Gew.-%, bevorzugt 2 Gew.-% bis 4 Gew.-% Citrat;
d) 0,4 Gew.-% bis 1,0 Gew.-%, bevorzugt 0,6 Gew.-% bis 0,8 Gew.-% mindestens eines Phosphonats; und
e) optional 3 Gew.-% bis 9 Gew.-%, bevorzugt 5 Gew.-% bis 7 Gew.-% 1,2-Propandiol.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine Lipase, sowie deren Gemische. Weitere Enzyme sind in dem Mittel vorteilhafterweise jeweils in einer Menge von 1 × 10⁻⁸ bis 5 Gewichts-Prozent bezogen auf aktives Protein enthalten. Zunehmend bevorzugt ist jedes weitere Enzym in einer Menge von 1 × 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv wird, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

In verschieden Ausführungsformen zeichnet sich das oben beschriebene Verfahren dadurch aus, dass die Protease bei einer Temperatur von 0-100°C, bevorzugt 0-60°C, weiter bevorzugt 20-50°C und am meisten bevorzugt bei ungefähr 40°C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar.

Alle Sachverhalte, Gegenstände und Ausführungsformen, die für die erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da die erfindungsgemäße Protease natürlicherweise bereits eine hydrolytische Aktivität besitzt und diese auch in Medien entfaltet, die sonst keine Reinigungskraft besitzen wie beispielsweise in blo-ßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Proteasen in Wasch- oder Reinigungsmitteln, beispielsweise wie oben beschrieben, zur (verbesserten) Entfernung von fetthaltigen Anschmutzungen, beispielsweise von Textilien oder harten Oberflächen.

Alle Sachverhalte, Gegenstände und Ausführungsformen, die für die erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1: Sequenz von Purafact Prime (SEQ ID NO:1) und einer Variante (SEQ ID NO:2) hiervon

Der Vergleich der Sequenzen von SEQ ID NO:1 (Referenz) und SEQ ID NO:2 (Mutante) zeigt, dass die Mutante 97% Identität gegenüber der Sequenz von SEQ ID NO:1 aufweist. Es sind folgende Mutationen in Bezug auf die Sequenz von SEQ ID NO:1 vorhanden: S24G, S33T, S53G, S78N, S101N, G128A und L217Q.

### Beispiel 2: Aktivitätsbestimmung der Protease

Die proteolytische Aktivität wurde durch einen diskontinuierlichen Assay unter Verwendung von Casein als Substrat bestimmt. Die Endkonzentrationen der Substratlösung waren 12 mg · ml⁻¹ Casein (hergestellt gemäß Hammarsten; Merck, Darmstadt, # 2242) und 30 mM Tris in synthetischem Leitungswasser. Synthetisches Leitungswasser ist eine Lösung von 0,029% (w/v) CaCl₂.2H₂Q, 0,014% (w/v) MgCl₂.6H₂O und 0,021% (w/v) NaHCOs mit einer Härte von 15° dH (deutsche Härte). Die Substratlösung wurde auf 70° C erhitzt und der pH-Wert wurde auf 8,5 bei 50° C unter Verwendung von 0,1 N NaOH eingestellt. Die Protease-Lösung wurde mit 2% (w/v) wasserfreiem Pentanatriumtriphosphat in synthetischem Leitungswasser, das mit Salzsäure auf pH 8,5 eingestellt wurde, hergestellt. Zu einer 600 µl Casein-Substratlösung wurden 200 µl der Enzymlösung gegeben. Das Gemisch wurde bei 50° C für 15 Minuten inkubiert. Die Reaktion wurde durch die Zugabe von 600 µl 0,44 M Trichloressigsäure (TCA), 0,22 M Natriumacetat in 3% (v/v) Eisessig beendet. Nach dem Abkühlen auf Eis für 15 Minuten wurde das TCA-unlösliche Protein durch Zentrifugation entfernt, ein Aliquot von 900 µl wurde mit 300 µl 2N NaOH gemischt und die Extinktion dieses Gemisches, die TCA-lösliche Peptide enthält, wurde bei 290 nm gemessen. Kontrollwerte wurden erhalten durch Zugabe von 600 µl TCA-Lösung zu 600 µl der Casein-Lösung gefolgt von 200 µl Enzymlösung. Eine Proteaselösung, die unter den Bedingungen dieses Assays eine Extinktionsänderung von 0,500 OD bei 290 nm aufweist, hat eine Aktivität von 10 HPE pro ml.

### Beispiel 3: Miniwaschtest

Der Miniwaschtest wurde mit dem Fermentationsüberstand der produzierten Protease durchgeführt. Bei den Referenzenzymen handelt es sich um technische Muster.
Bedingungen: 40°C, 16°dH Wasser, 1h
Enzymkonzentration: es wird AEP-gleich gewaschen (1,7 µg/mL)
Anschmutzungen:
   C-05 (Blut, Milch, Tusche)
   C-03 (Schokoladenmich und Ruß)
   EMPA 112 (Kakao)
   C-S-38 (Eigelb mit Pigment, gealtert)
   PC-10 (Erdnussöl, Pigment, Milch)

### Durchführung:

Ausgestanzte Gewebe (Durchmesser = 10 mm) in Mikrotiterplatte vorlegen, Waschlauge auf 40°C vortemperieren, Endkonzentration 4,52 g/L in 16°dH Wasser

Lauge und Enzym auf die Anschmutzung geben, für 1 h bei 40°C und 600 rpm inkubieren, anschließend die Anschmutzung mehrmals mit klarem Wasser spülen, trocken lassen und mit einem Farbmessgerät die Helligkeit bestimmen.

Je heller das Gewebe wird, desto besser ist die Reinigungsleistung. Gemessen wird hier der L-Wert = Helligkeit, je höher desto heller. Es wird mit einem gängigen Flüssigwaschmittel ohne Enzyme gewaschen (LSPA+).

### Probe 1: Waschmittel als Benchmark

Probe 2: Referenzprotease 1 (BLAP Wildtyp die Mutationen S3T, V4I, V193M, V199I und L211D in der Zählung gemäß BLAP)

Probe 3: Referenzprotease 2 (Subtilisin Carlsberg aus Bacillus licheniformis Stamm ATCC 53926) Probe 4: erfindungsgemäße Protease (SEQ ID NO:2)

In der Tabelle ist die Summe der Differenzen der L-Werte abgegeben. Dabei wurde die Leistung des Waschmittels (Probe 1) von den Proben mit Protease abgezogen.

**Tabelle 1:**

| | Probe 2 | Probe 3 | Probe 4 |
|---|---|---|---|
| Σ Δ L über 5 Anschmutzungen | 34,0 | 29,9 | 40,0 |

Wie aus der Tabelle 1 deutlich wird, weißt die Mutante gegenüber den Referenzenzymen eine verbesserte Waschleistung an den 5 ausgewählten Anschmutzungen auf.

### Beispiel 4:

### Verwendete Waschmittelmatrix:

| Chemischer Name | Gew.-% Aktivsubstanz im Rohmaterial | Gew.-% Aktivsubstanz in der Formulierung |
|---|---|---|
| Wasser demin. | 100 | Rest |
| Alkylbenzolsulfonsäure | 96 | 4,40 |
| Weitere anionische Tenside | 70 | 5,60 |
| C12-C18 Fettsäuren Na-Salz | 30 | 2,40 |
| Nicht-ionische Tenside | 100 | 4,40 |
| Phosphonate | 40 | 0,20 |
| Zitronensäure | 100 | 1,43 |
| NaOH | 50 | 0,95 |
| Entschäumer | t.q. | 0,01 |
| Glycerin | 100 | 2,00 |
| Konservierungsmittel | 100 | 0,08 |
| Ethanol | 93 | 1,00 |

| | | |
|---|---|---|
| Ohne opt. Aufheller, Parfüm, Farbstoff und Enzyme. | | |

## Patentansprüche

1. Protease umfassend eine Aminosäuresequenz wie in SEQ ID NO:2 angegeben.

2. Verfahren zur Herstellung einer Protease nach Anspruch 1, umfassend das Substituieren der Aminosäuren an den Positionen, die Positionen 24, 33, 53, 78, 101, 128 und 217 in SEQ ID NO:1 entsprechen, in einer Ausgangsprotease mit einer wie in SEQ ID NO:1 angegebenen Aminosäuresequenz , derart, dass die Protease an den entsprechenden Positionen die Aminosäuren 24G, 33T, 53G, 78N, 101N, 128A und 217Q umfasst.

3. Nukleinsäure kodierend für eine Protease nach Anspruch 1 oder kodierend für eine nach einem Verfahren nach Anspruch 2 erhältlichen Protease.

4. Vektor enthaltend eine Nukleinsäure nach Anspruch 3, insbesondere ein Klonierungsvektor oder ein Expressionsvektor.

5. Nicht menschliche Wirtszelle, die eine Nukleinsäure nach Anspruch 3 oder einen Vektor nach Anspruch 4 beinhaltet, oder die eine Protease nach Anspruch 1 beinhaltet, oder die eine nach einem Verfahren nach Anspruch 2 erhältliche Protease beinhaltet.

6. Verfahren zur Herstellung einer Protease umfassend
a) Kultivieren einer Wirtszelle gemäß Anspruch 5
b) Isolieren der Protease aus dem Kulturmedium oder aus der Wirtszelle.

7. Mittel, insbesondere ein Wasch- oder Reinigungsmittel, **dadurch gekennzeichnet, dass** es mindestens eine Protease nach Anspruch 1 oder eine nach einem Verfahren nach Anspruch 2 erhältliche Protease enthält, wobei die Protease bevorzugt in einer Menge bis zu 1 Gew.-%, besonders bevorzugt zwischen 0,006 Gew.-% bis 0,6 Gew.-%, bezogen auf aktives Protein enthalten ist.

8. Mittel nach Anspruch 7, **dadurch gekennzeichnet, dass** das Mittel ein Flüssigwaschmittel ist, das anionische Tenside, insbesondere lineare Alkylbenzolsulfonate, nicht-ionische Tenside, und mindestens einen Builder umfasst.

9. Mittel nach Anspruch 8, wobei das Mittel jeweils bezogen auf das Gesamtgewicht des Mittels umfasst:
a) 4 Gew.-% bis 10 Gew.-%, bevorzugt 6 Gew.-% bis 8 Gew.-% eines linearen Alkylbenzolsulfonats;
b) 6 Gew.-% bis 12 Gew.-%, bevorzugt 8 Gew.-% bis 10 Gew.-% eines weiteren anionischen Tensids, insbesondere eine Alkylethersulfats;
c) 0,5 Gew.-% bis 6 Gew.-%, bevorzugt 2 Gew.-% bis 4 Gew.-% Citrat;
d) 0,4 Gew.-% bis 1,0 Gew.-%, bevorzugt 0,6 Gew.-% bis 0,8 Gew.-% mindestens eines Phosphonats; und
e) optional 3 Gew.-% bis 9 Gew.-%, bevorzugt 5 Gew.-% bis 7 Gew.-% 1,2-Propandiol.

10. Verfahren zur Reinigung von Textilien oder harten Oberflächen, **dadurch gekennzeichnet, dass** in mindestens einem Verfahrensschritt ein Mittel nach einem der Ansprüche 7 bis 9 angewendet wird, oder dass in mindestens einem Verfahrensschritt eine Protease nach Anspruch 1 oder eine nach einem Verfahren nach Anspruch 2 erhältliche Protease angewendet wird.

11. Verwendung einer Protease nach Anspruch 1 oder einer nach einem Verfahren nach Anspruch 2 erhältliche Protease in einem Wasch- oder Reinigungsmittel zur Entfernung von proteinhaltigen Anschmutzungen.

## Claims

1. A protease comprising an amino acid sequence as set forth in SEQ ID NO:2.

2. A method of producing a protease according to claim 1, comprising substituting the amino acids at the positions corresponding to positions 24, 33, 53, 78, 101, 128 and 217 in SEQ ID NO:1 in a starting protease having an amino acid sequence as set forth in SEQ ID NO:1, such that the protease comprises the amino acids 24G, 33T, 53G, 78N, 101N, 128A and 217Q at the corresponding positions.

3. A nucleic acid encoding a protease according to claim 1 or encoding a protease obtainable by a method according to claim 2.

4. A vector containing a nucleic acid according to claim 3, in particular a cloning vector or an expression vector.

5. A non-human host cell comprising a nucleic acid according to claim 3 or a vector according to claim 4, or comprising a protease according to claim 1, or comprising a protease obtainable by a method according to claim 2.

6. A process for producing a protease comprising
a) Culturing a host cell according to claim 5
b) Isolating the protease from the culture medium or from the host cell.

7. An agent, in particular a detergent or cleaning agent, **characterized in that** it contains at least one protease according to claim 1 or a protease obtainable by a method according to claim 2, the protease preferably being present in an amount of up to 1 wt.%, particularly preferably between 0.006 wt.% and 0.6 wt.%, based on active protein.

8. The agent according to claim 7, **characterized in that** the agent is a liquid detergent comprising anionic surfactants, in particular linear alkyl benzene sulfonates, nonionic surfactants, and at least one builder.

9. The agent according to claim 8, wherein the agent comprises, each based on the total weight of the agent:
a) 4 wt.% to 10 wt.%, preferably 6 wt.% to 8 wt.%, of a linear alkyl benzene sulfonate;
b) 6 wt.% to 12 wt.%, preferably 8 wt.% to 10 wt.%, of a further anionic surfactant, in particular an alkyl ether sulfate;
c) 0.5 wt.% to 6 wt.%, preferably 2 wt.% to 4 wt.% citrate;
d) 0.4 wt.% to 1.0 wt.%, preferably 0.6 wt.% to 0.8 wt.%, of at least one phosphonate; and
e) optionally from 3 wt.% to 9 wt.%, preferably from 5 wt.% to 7 wt.%, of 1,2-propanediol.

10. A method for cleaning textiles or hard surfaces, **characterized in that** in at least one method step an agent according to any one of claims 7 to 9 is applied, or that in at least one method step a protease according to claim 1 or a protease obtainable by a method according to claim 2 is applied.

11. Use of a protease according to claim 1 or a protease obtainable by a method according to claim 2 in a washing or cleaning composition for removing protein-containing soils.

## Revendications

1. Protéase comprenant une séquence d'acides aminés telle qu'indiquée dans SEQ ID NO:2.

2. Procédé de production d'une protéase selon la revendication 1, qui comprend la substitution des acides aminés aux positions correspondant aux positions 24, 33, 53, 78, 101, 128 et 217 dans SEQ ID NO:1 dans une protéase de départ ayant une séquence d'acides aminés telle qu'indiquée dans SEQ ID NO:1, de telle sorte que la protéase aux positions correspondantes comprenne les acides aminés 24G, 33T, 53G, 78N, 101N, 128A et 217Q.

3. Acide nucléique codant pour une protéase selon la revendication 1 ou codant pour une protéase pouvant être obtenue par un procédé selon la revendication 2.

4. Vecteur contenant un acide nucléique selon la revendication 3, en particulier un vecteur de clonage ou un vecteur d'expression.

5. Cellule hôte non humaine qui comprend un acide nucléique selon la revendication 3 ou un vecteur selon la revendication 4, ou qui comprend une protéase selon la revendication 1, ou qui comprend une protéase pouvant être obtenue par un procédé selon la revendication 2.

6. Procédé de production d'une protéase comprenant
a) Culture d'une cellule hôte selon la revendication 5
b) Isoler la protéase du milieu de culture ou de la cellule hôte.

7. Produit, en particulier un produit de lavage ou de nettoyage, **caractérisé en ce qu'**il contient au moins une protéase selon la revendication 1 ou une protéase pouvant être obtenue par un procédé selon la revendication 2, la protéase étant contenue de préférence en une quantité allant jusqu'à 1 % en poids, de manière particulièrement préférée entre 0,006 % en poids et 0,6 % en poids, par rapport à la protéine active.

8. Agent selon la revendication 7, **caractérisé en ce que** l'agent est un détergent liquide qui comprend des tensioactifs anioniques, en particulier des alkylbenzènesulfonates linéaires, des tensioactifs non ioniques, et au moins un adjuvant.

9. Agent selon la revendication 8, dans lequel l'agent comprend chaque fois par rapport au poids total de l'agent :
a) 4 % en poids à 10 % en poids, de préférence 6 % en poids à 8 % en poids d'un alkylbenzènesulfonate linéaire;
b) 6 % en poids à 12 % en poids, de préférence 8 % en poids à 10 % en poids d'un autre agent tensioactif anionique, en particulier un alkyléthersulfate;
c) 0,5 % à 6 % en poids, de préférence 2 % à 4 % en poids de citrate;
d) 0,4 % à 1,0 % en poids, de préférence 0,6 % à 0,8 % en poids d'au moins un phosphonate; et
e) facultativement, de 3 % à 9 % en poids, de préférence de 5 % à 7 % en poids de 1,2-propanediol.

10. Procédé de nettoyage de textiles ou de surfaces dures, **caractérisé en ce que** dans au moins une étape du procédé, on utilise un agent selon l'une des revendications 7 à 9, ou **en ce que** dans au moins une étape du procédé, on utilise une protéase selon la revendication 1 ou une protéase pouvant être obtenue par un procédé selon la revendication 2.

11. Utilisation d'une protéase selon la revendication 1 ou d'une protéase pouvant être obtenue par un procédé selon la revendication 2 dans un produit de lavage ou de nettoyage pour éliminer des salissures contenant des protéines.
